# EUROPEAN PATENT SPECIFICATION

(11) **EP 1 357 928 B1**
(45) Date of publication and mention of the grant of the patent: **16.11.2011**
(21) Application number: 01273387.9
(22) Date of filing: 16.11.2001
(51) Int. Cl.: A61K 47/48

(54) **A NOVEL PHARMACEUTICAL COMPOUND AND METHODS OF MAKING AND USING SAME**
NEUARTIGE PHARMAZEUTISCHE VERBINDUNG UND VERFAHREN ZUR HERSTELLUNG UND VERWENDUNG DERSELBEN
NOUVEAU COMPOSÉ PHARMACEUTIQUE ET PROCÉDÉS DE FABRICATION ET D'UTILISATION DE CE COMPOSÉ

(30) Priority: 16.11.2000 US 248705 P; 16.11.2000 US 248607 P; 16.11.2000 US 248611 P; 16.11.2000 US 248609 P; 16.11.2000 US 248608 P; 16.11.2000 US 248606 P; 16.11.2000 US 248604 P; 16.11.2000 US 248603 P; 16.11.2000 US 248601 P; 16.11.2000 US 248600 P; 16.11.2000 US 248712 P; 16.11.2000 US 248711 P; 16.11.2000 US 248709 P; 16.11.2000 US 248708 P; 16.11.2000 US 248707 P; 16.11.2000 US 248706 P; 16.11.2000 US 248704 P; 16.11.2000 US 248703 P; 16.11.2000 US 248702 P; 16.11.2000 US 248701 P; 16.11.2000 US 248698 P; 16.11.2000 US 248697 P; 16.11.2000 US 248696 P; 16.11.2000 US 248695 P; 16.11.2000 US 248694 P; 16.11.2000 US 248693 P; 16.11.2000 US 248692 P; 16.11.2000 US 248691 P; 16.11.2000 US 248710 P; 16.11.2000 US 248689 P; 16.11.2000 US 248688 P; 16.11.2000 US 248686 P; 16.11.2000 US 248720 P; 16.11.2000 US 248719 P; 16.11.2000 US 248718 P; 16.11.2000 US 248717 P; 16.11.2000 US 248716 P; 16.11.2000 US 248715 P; 16.11.2000 US 248714 P; 16.11.2000 US 248713 P; 16.11.2000 US 248536 P; 16.11.2000 US 248535 P; 16.11.2000 US 248733 P; 16.11.2000 US 248732 P; 16.11.2000 US 248731 P; 16.11.2000 US 248729 P; 16.11.2000 US 248728 P; 16.11.2000 US 248530 P; 16.11.2000 US 248730 P; 16.11.2000 US 248727 P; 16.11.2000 US 248700 P; 16.11.2000 US 248699 P; 16.11.2000 US 248726 P; 16.11.2000 US 248725 P; 16.11.2000 US 248724 P; 16.11.2000 US 248723 P; 16.11.2000 US 248722 P; 16.11.2000 US 248721 P; 16.11.2000 US 248540 P; 16.11.2000 US 248539 P; 16.11.2000 US 248538 P; 16.11.2000 US 248537 P; 16.11.2000 US 248533 P; 16.11.2000 US 248532 P; 16.11.2000 US 248531 P; 16.11.2000 US 248529 P; 16.11.2000 US 248528 P; 16.11.2000 US 248527 P; 16.11.2000 US 248526 P; 16.11.2000 US 248525 P; 16.11.2000 US 248524 P; 16.11.2000 US 248670 P; 16.11.2000 US 248789 P; 16.11.2000 US 248599 P; 16.11.2000 US 248745 P; 16.11.2000 US 248746 P; 16.11.2000 US 248747 P; 16.11.2000 US 248748 P; 16.11.2000 US 248744 P; 16.11.2000 US 248743 P; 16.11.2000 US 248756 P; 16.11.2000 US 248602 P; 16.11.2000 US 248598 P; 16.11.2000 US 248597 P; 16.11.2000 US 248596 P; 16.11.2000 US 248595 P; 16.11.2000 US 248594 P; 16.11.2000 US 248858 P; 16.11.2000 US 248857 P; 16.11.2000 US 248856 P; 16.11.2000 US 248855 P; 16.11.2000 US 248854 P; 16.11.2000 US 248853 P; 16.11.2000 US 248852 P; 16.11.2000 US 248851 P; 16.11.2000 US 248850 P; 16.11.2000 US 248849 P; 16.11.2000 US 248848 P; 16.11.2000 US 248792 P; 16.11.2000 US 248790 P; 16.11.2000 US 248669 P; 16.11.2000 US 248668 P; 16.11.2000 US 248667 P; 16.11.2000 US 248666 P; 16.11.2000 US 248665 P; 16.11.2000 US 248664 P; 16.11.2000 US 248793 P; 16.11.2000 US 248791 P; 16.11.2000 US 248684 P; 16.11.2000 US 248683 P; 16.11.2000 US 248682 P; 16.11.2000 US 248681 P; 16.11.2000 US 248680 P; 16.11.2000 US 248671 P; 16.11.2000 US 248679 P; 16.11.2000 US 248675 P; 16.11.2000 US 248676 P; 16.11.2000 US 248677 P; 16.11.2000 US 248678 P; 16.11.2000 US 248673 P; 16.11.2000 US 248674 P; 16.11.2000 US 248672 P; 16.11.2000 US 248784 P; 16.11.2000 US 248785 P; 16.11.2000 US 248786 P; 16.11.2000 US 248775 P; 16.11.2000 US 248773 P; 16.11.2000 US 248766 P; 16.11.2000 US 248765 P; 16.11.2000 US 248833 P; 16.11.2000 US 248783 P; 16.11.2000 US 248781 P; 16.11.2000 US 248780 P; 16.11.2000 US 248778 P; 16.11.2000 US 248767 P; 16.11.2000 US 248787 P; 16.11.2000 US 248774 P; 16.11.2000 US 248764 P; 16.11.2000 US 248782 P; 16.11.2000 US 248779 P; 16.11.2000 US 248685 P; 16.11.2000 US 248772 P; 16.11.2000 US 248771 P; 16.11.2000 US 248777 P; 16.11.2000 US 248776 P; 16.11.2000 US 248770 P; 16.11.2000 US 248768 P; 16.11.2000 US 248769 P; 16.11.2000 US 248796 P; 16.11.2000 US 248797 P; 16.11.2000 US 248795 P; 16.11.2000 US 248794 P; 16.11.2000 US 248663 P; 16.11.2000 US 248662 P; 16.11.2000 US 248660 P; 16.11.2000 US 248659 P; 16.11.2000 US 248658 P; 16.11.2000 US 248656 P; 16.11.2000 US 248654 P; 16.11.2000 US 248653 P; 16.11.2000 US 248651 P; 16.11.2000 US 248650 P; 16.11.2000 US 248648 P; 16.11.2000 US 248647 P; 16.11.2000 US 248645 P; 16.11.2000 US 248643 P; 16.11.2000 US 248642 P; 16.11.2000 US 248640 P; 16.11.2000 US 248637 P; 16.11.2000 US 248636 P; 16.11.2000 US 248634 P; 16.11.2000 US 248632 P; 16.11.2000 US 248631 P; 16.11.2000 US 248630 P; 16.11.2000 US 248629 P; 16.11.2000 US 248627 P; 16.11.2000 US 248625 P; 16.11.2000 US 248763 P; 16.11.2000 US 248761 P; 16.11.2000 US 248759 P; 16.11.2000 US 248757 P; 16.11.2000 US 248754 P; 16.11.2000 US 248753 P; 16.11.2000 US 248749 P; 16.11.2000 US 248616 P; 16.11.2000 US 248615 P; 16.11.2000 US 248614 P; 16.11.2000 US 248613 P; 16.11.2000 US 248612 P; 16.11.2000 US 248605 P; 16.11.2000 US 248610 P; 16.11.2000 US 248661 P; 16.11.2000 US 248657 P; 16.11.2000 US 248655 P; 16.11.2000 US 248652 P; 16.11.2000 US 248649 P; 16.11.2000 US 248646 P; 16.11.2000 US 248644 P; 16.11.2000 US 248641 P; 16.11.2000 US 248639 P; 16.11.2000 US 248638 P; 16.11.2000 US 248635 P; 16.11.2000 US 248633 P; 16.11.2000 US 248628 P; 16.11.2000 US 248626 P; 16.11.2000 US 248624 P; 16.11.2000 US 248762 P; 16.11.2000 US 248760 P; 16.11.2000 US 248758 P; 16.11.2000 US 248755 P; 16.11.2000 US 248752 P; 16.11.2000 US 248751 P; 16.11.2000 US 248750 P; 16.11.2000 US 248742 P; 16.11.2000 US 248741 P; 16.11.2000 US 248740 P; 16.11.2000 US 248739 P; 16.11.2000 US 248736 P; 16.11.2000 US 248735 P; 16.11.2000 US 248734 P; 16.11.2000 US 248623 P; 16.11.2000 US 248622 P; 16.11.2000 US 248621 P; 16.11.2000 US 248738 P; 16.11.2000 US 248737 P; 16.11.2000 US 248620 P; 16.11.2000 US 248619 P; 16.11.2000 US 248618 P; 16.11.2000 US 248617 P
(43) Date of publication of application: 05.11.2003
(73) Proprietor: Shire LLC, Florence, KY 41042 (US)
(72) Inventor: PICARIELLO, Thomas, Blacksburg, VA 24060 (US)
(74) Representative: Atkinson, Jonathan David Mark
(86) International application number: PCT/US2001/043117
(87) International publication number: WO 2003/020200

(56) References cited:
- WO-A-00/37103
- WO-A-00/52078
- WO-A-02/34237
- WO-A-94/11021
- US-A- 4 427 660
- US-A- 4 801 575
- US-A- 4 902 505
- US-A- 5 534 496
- US-A- 5 846 743
- US-A- 6 030 941
- US-A- 6 048 736

## Description

### FIELD OF INVENTION:

The present invention relates to a novel pharmaceutical compound that comprises a polypeptide that is preferably covalently attached to oxycodone and acetaminophen, as well as methods for protecting and administering oxycodone and acetaminophen. This novel compound, referred to as a CARRIERWAVE^{™} Molecular Analogue (CMA), has the benefit of taking a known effective pharmaceutical agent that is both well studied and occupies a known segment of the pharmaceutical market, and combining it with a carrier compound that enhances the usefulness of the pharmaceutical agent without compromising its pharmaceutical effectiveness.

The present invention relates to a novel pharmaceutical compound that comprises a polypeptide that is preferably covalently attached to oxycodone, as well as methods for protecting and administering oxycodone. This novel compound, referred to as a CARRIERWAVE^{™} Molecular Analogue (CMA), has the benefit of taking a known effective pharmaceutical agent that is both well studied and occupies a known segment of the pharmaceutical market, and combining it with a carrier compound that enhances the usefulness of the pharmaceutical agent without compromising its pharmaceutical effectiveness.

### BACKGROUND OF THE INVENTION:

Oxycodone and acetaminophen are used together in the treatment of pain. The chemical name of acetaminophen is N-acetyl-p-aminophenol. The structure of oxycodone is:

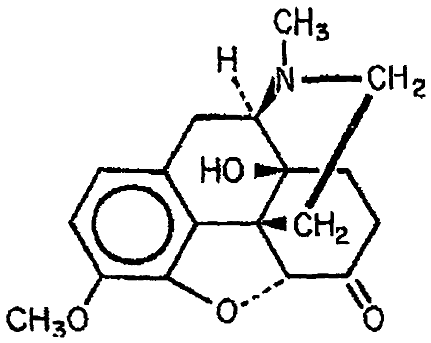

The novel pharmaceutical compound of the present invention is useful in accomplishing one or more of the following goals: enhancement of the chemical stability of the original compound; alteration of the release profile of an orally administered product; enhanced digestion or absorption; targeted delivery to particular tissue/cell type; and provision for an oral dosage form when none exists. The novel pharmaceutical compound may contain one or more of the following: another active pharmaceutical agent, an adjuvant, or an inhibitor.

Active agent delivery systems are often critical for the effective delivery of a biologically active agent (active agent) to the appropriate target. The importance of these systems becomes magnified when patient compliance and active agent stability are taken under consideration. For instance, one would expect patient compliance to increase markedly if an active agent is administered orally in lieu of an injection or another invasive technique. Increasing the stability of the active agent, such as prolonging shelf life or survival in the stomach, will assure dosage reproducibility and perhaps even reduce the number of dosages required which could improve patient compliance.

Absorption of an orally administered active agent is often blocked by the harshly acidic stomach milieu, powerful digestive enzymes in the GI tract, permeability of cellular membranes and transport across lipid bilayers. Incorporating adjuvants such as resorcinol, surfactants, polyethylene glycol (PEG) or bile acids enhance permeability of cellular membranes. Microencapsulating active agents using protenoid microspheres, liposomes or polysaccharides have been effective in abating enzyme degradation of the active agent. Enzyme inhibiting adjuvants have also been used to prevent enzyme degradation. Enteric coatings have been used as a protector of pharmaceuticals in the stomach.

Active agent delivery systems also provide the ability to control the release of the active agent. For example, formulating diazepam with a copolymer of glutamic acid and aspartic acid enables a sustained release of the active agent. As another example, copolymers of lactic acid and glutaric acid are used to provide timed release of human growth hormone. A wide range of pharmaceuticals purportedly provide sustained release through microencapsulation of the active agent in amides of dicarboxylic acids, modified amino acids or thermally condensed amino acids. Slow release rendering additives can also be intermixed with a large array of active agents in tablet formulations.

Each of these technologies imparts enhanced stability and time-release properties to active agent substances. Unfortunately, these technologies suffer from several shortcomings. Incorporation of the active agent is often dependent on diffusion into the microencapsulating matrix, which may not be quantitative and may complicate dosage reproducibility. In addition, encapsulated drugs rely on diffusion out of the matrix, which is highly dependant on the water solubility of the active agent. Conversely, water-soluble microspheres swell by an infinite degree and, unfortunately, may release the active agent in bursts with little active agent available for sustained release. Furthermore, in some technologies, control of the degradation process required for active agent release is unreliable. For example, an enterically coated active agent depends on pH to release the active agent and, as such, is difficult to control the rate of release.

In the past, use has been made of amino acid side chains of polypeptides as pendant groups to which active agents can be attached. These technologies typically require the use of spacer groups between the amino acid pendant group and the active agent. The peptide-drug conjugates of this class of drug delivery system rely on enzymes in the bloodstream for the release of the drug and, as such, are not used for oral administration. Examples of timed and targeted release of injectable or subcutaneous pharmaceuticals include: linking of norethindrone, via a hydroxypropyl spacer, to the gamma carboxylate of polyglutamic acid; and linking of nitrogen mustard, via a peptide spacer, to the gamma carbamide of polyglutamine. Dexamethasone has been covalently attached directly to the beta carboxylate of polyaspartic acid without a spacer group.
This prodrug formulation was designed as a colon-specific drug delivery system where the drug is released by bacterial hydrolytic enzymes residing in the large intestines. The released dexamethasone active agent, in turn, was targeted to treat large bowel disorders and was not intended to be absorbed into the bloodstream. Yet another technology combines the advantages of covalent drug attachment with liposome formation where the active ingredient is attached to highly ordered lipid films (known as HARs) via a peptide linker. Thus, there has been no drug delivery system, heretofore reported, that incorporates the concept of attaching an active ingredient to a polypeptide pendant group with its targeted delivery into the bloodstream via oral administration.

It is also important to control the molecular weight, molecular size and particle size of the active agent delivery system. Variable molecular weights have unpredictable diffusion rates and pharmacokinetics. High molecular weight carriers are digested slowly or late, as in the case of naproxen-linked dextran, which is digested almost exclusively in the colon by bacterial enzymes. High molecular weight microspheres usually have high moisture content which may present a problem with water labile active ingredients. Particle size not only becomes a problem with injectable drugs, as in the HAR application, but absorption through the brush-border membrane of the intestines is limited to less than 5 microns.

Oxycodone is a known pharmaceutical agent that is used in the treatment of pain. The structure of oxycodone is:

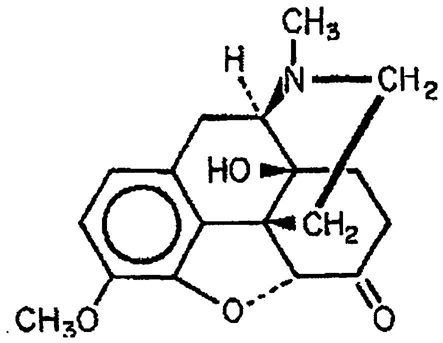

The novel pharmaceutical compound of the present invention is useful in accomplishing one or more of the following goals: enhancement of the chemical stability of the original compound; alteration of the release profile of an orally administered product; enhanced digestion or absorption; targeted delivery to particular tissue/cell type; and provision for an oral dosage form when none exists. The novel pharmaceutical compound may contain one or more of the following: another active pharmaceutical agent, an adjuvant, or an inhibitor.

Active agent delivery systems are often critical for the effective delivery of a biologically active agent (active agent) to the appropriate target. The importance of these systems becomes magnified when patient compliance and active agent stability are taken under consideration. For instance, one would expect patient compliance to increase markedly if an active agent is administered orally in lieu of an injection or another invasive technique. Increasing the stability of the active agent, such as prolonging shelf life or survival in the stomach, will assure dosage reproducibility and perhaps even reduce the number of dosages required which could improve patient compliance.

Absorption of an orally administered active agent is often blocked by the harshly acidic stomach milieu, powerful digestive enzymes in the GI tract, permeability of cellular membranes and transport across lipid bilayers. Incorporating adjuvants such as resorcinol, surfactants, polyethylene glycol (PEG) or bile acids enhance permeability of cellular membranes. Microencapsulating active agents using protenoid microspheres, liposomes or polysaccharides have been effective in abating enzyme degradation of the active agent. Enzyme inhibiting adjuvants have also been used to prevent enzyme degradation. Enteric coatings have been used as a protector of pharmaceuticals in the stomach.

Active agent delivery systems also provide the ability to control the release of the active agent. For example, formulating diazepam with a copolymer of glutamic acid and aspartic acid enables a sustained release of the active agent. As another example, copolymers of lactic acid and glutaric acid are used to provide timed release of human growth hormone. A wide range of pharmaceuticals purportedly provide sustained release through microencapsulation of the active agent in amides of dicarboxylic acids, modified amino acids or thermally condensed amino acids. Slow release rendering additives can also be intermixed with a large array of active agents in tablet formulations.

Each of these technologies imparts enhanced stability and time-release properties to active agent substances. Unfortunately, these technologies suffer from several shortcomings. Incorporation of the active agent is often dependent on diffusion into the microencapsulating matrix, which may not be quantitative and may complicate dosage reproducibility. In addition, encapsulated drugs rely on diffusion out of the matrix, which is highly dependant on the water solubility of the active agent. Conversely, water-soluble microspheres swell by an infinite degree and, unfortunately, may release the active agent in bursts with little active agent available for sustained release. Furthermore, in some technologies, control of the degradation process required for active agent release is unreliable. For example, an enterically coated active agent depends on pH to release the active agent and, as such, is difficult to control the rate of release.

In the past, use has been made of amino acid side chains of polypeptides as pendant groups to which active agents can be attached. These technologies typically require the use of spacer groups between the amino acid pendant group and the active agent. The peptide-drug conjugates of this class of drug delivery system rely on enzymes in the bloodstream for the release of the drug and, as such, are not used for oral administration. Examples of timed and targeted release of injectable or subcutaneous pharmaceuticals include: linking of norethindrone, via a hydroxypropyl spacer, to the gamma carboxylate of polyglutamic acid; and linking of nitrogen mustard, via a peptide spacer, to the gamma carbamide of polyglutamine. Dexamethasone has been covalently attached directly to the beta carboxylate of polyaspartic acid without a spacer group. This prodrug formulation was designed as a colon-specific drug delivery system where the drug is released by bacterial hydrolytic enzymes residing in the large intestines. The released dexamethasone active agent, in turn, was targeted to treat large bowel disorders and was not intended to be absorbed into the bloodstream. Yet another technology combines the advantages of covalent drug attachment with liposome formation where the active ingredient is attached to highly ordered lipid films (known as HARs) via a peptide linker. Thus, there has been no drug delivery system, heretofore reported, that incorporates the concept of attaching an active ingredient to a polypeptide pendant group with its targeted delivery into the bloodstream via oral administration.

It is also important to control the molecular weight, molecular size and particle size of the active agent delivery system. Variable molecular weights have unpredictable diffusion rates and pharmacokinetics. High molecular weight carriers are digested slowly or late, as in the case of naproxen-linked dextran, which is digested almost exclusively in the colon by bacterial enzymes. High molecular weight microspheres usually have high moisture content which may present a problem with water labile active ingredients. Particle size not only becomes a problem with injectable drugs, as in the HAR application, but absorption through the brush-border membrane of the intestines is limited to less than 5 microns.

WO 94/11021 discloses pharmaceutically-active heterodimers comprising a bradykinin antagonist component covalently linked to another different pharmacophore component. These are obtained by linking a bradykinin antagonist peptide to another pharmcophore which is not a bradykinin antagonist.

WO 00/37103 discloses compounds which can be used to selectively deliver moieties to nerve cells. The compounds include a binding agent capable of selectively binding to nerve cell surface receptors, a linking group and a moiety which performs a non-cytotoxic function when absorbed by a nerve cell.

### SUMMARY OF THE INVENTION:

The present invention provides covalent attachment of the active agent (oxycodone and acetaminophen) to a polymer of peptides or amino acids. The invention is distinguished from the above-mentioned technologies by virtue of covalently attaching oxycodone and acetaminophen to the N-terminus, the C-terminus or directly to the amino acid side chain of an oligopeptide or polypeptide, also referred to herein as a carrier peptide. In certain applications, the polypeptide will stabilize the active agent, primarily in the stomach, through conformational protection. In these applications, delivery of the active agent is controlled, in part, by the kinetics of unfolding of the carrier peptide. Upon entry into the upper intestinal tract, indigenous enzymes release the active ingredient for absorption by the body by selectively hydrolyzing the peptide bonds of the carrier peptide. This enzymatic action introduces a second order sustained release mechanism.

Alternatively, the present invention provides a pharmaceutical composition comprising oxycodone and acetaminophen microencapsulated by a polypeptide.

The invention provides a composition comprising a polypeptide and oxycodone and acetaminophen covalently attached to the polypeptide. Preferably, the polypeptide is (i) an oligopeptide, (ii) a homopolymer of one of the twenty naturally occurring amino acids, or (iii) a heteropolymer of two or more naturally occurring amino acids.

Oxycodone and acetaminophen preferably is covalently attached to a side chain, the N-terminus or the C-terminus of the polypeptide. In another preferred embodiment, the active agent is an amine and is covalently attached to the C-terminus of the polypeptide. In another preferred embodiment, the active agent is an alcohol and is covalently attached to the C-terminus of the polypeptide. In yet another preferred embodiment, the active agent is an alcohol and is covalently attached to the N-terminus of the polypeptide.

The composition of the invention can also include one or more of a microencapsulating agent, an adjuvant and a pharmaceutically acceptable excipient. The microencapsulating agent can be selected from polyethylene glycol (PEG), an amino acid, a sugar and a salt. When an adjuvant is included in the composition, the adjuvant preferably activates an intestinal transporter.

Preferably, the composition of the invention is in the form of an ingestable tablet, an intravenous preparation or an oral suspension. The active agent can be conformationally protected by folding of the polypeptide about the active agent. In another embodiment, the polypeptide is capable of releasing the active agent from the composition in a pH-dependent manner.

The disclosure also provides a method for protecting oxycodone and acetaminophen from degradation comprising covalently attaching it to a polypeptide.

The disclosure also provides a method for delivering oxycodone and acetaminophen to a patient, the patient being a human or a non-human animal, comprising administering to the patient a composition comprising a polypeptide and an active agent covalently attached to the polypeptide. In a preferred embodiment, oxycodone and acetaminophen are released from the composition by an enzyme-catalyzed release. In another preferred embodiment, oxycodone and acetaminophen are released in a time-dependent manner based on the pharmacokinetics of the enzyme-catalyzed release. In another preferred embodiment, the composition further comprises a microencapsulating agent and oxycodone and acetaminophen are released from the composition by dissolution of the microencapsulating agent. In another preferred embodiment, oxycodone and acetaminophen are released from the composition by a pH-dependent unfolding of the polypeptide. In another preferred embodiment, oxycodone and acetaminophen are released from the composition in a sustained release. In yet another preferred embodiment, the composition further comprises an adjuvant covalently attached to the polypeptide and release of the adjuvant from the composition is controlled by the polypeptide. The adjuvant can be microencapsulated into a carrier peptide-drug conjugate for biphasic release of active ingredients.

The invention also provides a method for preparing a composition comprising a polypeptide and an active agent covalently attached to the polypeptide. The method comprises the steps of:
(a) attaching oxycodone and acetaminophen to a side chain of an amino acid to form an active agent/amino acid complex;
(b) forming an active agent/amino acid complex N-carboxyanhydride (NCA) from the active agent/amino acid complex; and
(c) polymerizing the active agent/amino acid complex N-carboxyanhydride (NCA).

In a preferred embodiment, steps (a) and (b) are repeated prior to step (c) with a second active agent. When steps (a) and (b) are repeated prior to step (c) with a second agent, oxycodone and acetaminophen and a second active agent can be copolymerized in step (c). In another preferred embodiment, the amino acid is glutamic acid and the active agent is released from the glutamic acid as a dimer upon a hydrolysis of the polypeptide and wherein the active agent is released from the glutamic acid by coincident intramolecular transamination. In another preferred embodiment, the glutamic acid is replaced by an amino acid selected from the group consisting of aspartic acid, arginine, asparagine, cysteine, lysine, threonine, and serine, and wherein the active agent is attached to the side chain of the amino acid to form an amide, a thioester, an ester, an ether, a urethane, a carbonate, an anhydride or a carbamate.

It is to be understood that both the foregoing general description and the following detailed description are exemplary, but are not restrictive, of the invention.

The present invention provides covalent attachment of the active agent (oxycodone) to a polymer of peptides or amino acids. The invention is distinguished from the above-mentioned technologies by virtue of covalently attaching oxycodone to the N-terminus, the C-terminus or directly to the amino acid side chain of an oligopeptide or polypeptide, also referred to herein as a carrier peptide. In certain applications, the polypeptide will stabilize the active agent, primarily in the stomach, through conformational protection. In these applications, delivery of the active agent is controlled, in part, by the kinetics of unfolding of the carrier peptide. Upon entry into the upper intestinal tract, indigenous enzymes release the active ingredient for absorption by the body by selectively hydrolyzing the peptide bonds of the carrier peptide. This enzymatic action introduces a second order sustained release mechanism.

Alternatively, the present invention provides a pharmaceutical composition comprising oxycodone microencapsulated by a polypeptide.

The invention provides a composition comprising a carrier polypeptide and oxycodone covalently attached to the carrier polypeptide. Preferably, the polypeptide is (i) an oligopeptide, (ii) a homopolymer of one of the twenty naturally occurring amino acids, or (iii) a heteropolymer of two or more naturally occurring amino acids.

Oxycodone preferably is covalently attached to a side chain, the N-terminus or the C-terminus of the polypeptide. In another preferred embodiment, the active agent is an amine and is covalently attached to the C-terminus of the polypeptide. In another preferred embodiment, the active agent is an alcohol and is covalently attached to the C-terminus of the polypeptide. In yet another preferred embodiment, the active agent is an alcohol and is covalently attached to the N-terminus of the polypeptide.

The composition of the invention can also include one or more of a microencapsulating agent, an adjuvant and a pharmaceutically acceptable excipient. The microencapsulating agent can be selected from polyethylene glycol (PEG), an amino acid, a sugar and a salt. When an adjuvant is included in the composition, the adjuvant preferably activates an intestinal transporter.

Preferably, the composition of the invention is in the form of an ingestable tablet, an intravenous preparation or an oral suspension. The active agent can be conformationally protected by folding of the polypeptide about the active agent. In another embodiment, the polypeptide is capable of releasing the active agent from the composition in a pH-dependent manner.

The dislosure also provides a method for protecting oxycodone from degradation comprising covalently attaching it to a polypeptide.

The disclosure also provides a method for delivering oxycodone to a patient, the patient being a human or a non-human animal, comprising administering to the patient a composition comprising a polypeptide and an active agent covalently attached to the polypeptide. In a preferred embodiment, oxycodone is released from the composition by an enzyme-catalyzed release. In another preferred embodiment, oxycodone is released in a time-dependent manner based on the pharmacokinetics of the enzyme-catalyzed release. In another preferred embodiment, the composition further comprises a microencapsulating agent and oxycodone is released from the composition by dissolution of the microencapsulating agent. In another preferred embodiment, oxycodone is released from the composition by a pH-dependent unfolding of the polypeptide. In another preferred embodiment, oxycodone is released from the composition in a sustained release. In yet another preferred embodiment, the composition further comprises an adjuvant covalently attached to the polypeptide and release of the adjuvant from the composition is controlled by the polypeptide. The adjuvant can be microencapsulated into a carrier peptide-drug conjugate for biphasic release of active ingredients.

The invention also provides a method for preparing a composition comprising a carrier polypeptide and an active agent covalently attached to the carrier polypeptide. The method comprises the steps of:
(a) attaching oxycodone to a side chain of an amino acid to form an active agent/amino acid complex;
(b) forming an active agent/amino acid complex N-carboxyanhydride (NCA) from the active agent/amino acid complex; and
(c) polymerizing the active agent/amino acid complex N-carboxyanhydride (NCA).

In a preferred embodiment, steps (a) and (b) are repeated prior to step (c) with a second active agent. When steps (a) and (b) are repeated prior to step (c) with a second agent, oxycodone and a second active agent can be copolymerized in step (c). In another preferred embodiment, the amino acid is glutamic acid and the active agent is released from the glutamic acid as a dimer upon a hydrolysis of the polypeptide and wherein the active agent is released from the glutamic acid by coincident intramolecular transamination. In another preferred embodiment, the glutamic acid is replaced by an amino acid selected from the group consisting of aspartic acid, arginine, asparagine, cysteine, lysine, threonine, and serine, and wherein the active agent is attached to the side chain of the amino acid to form an amide, a thioester, an ester, an ether, a urethane, a carbonate, an anhydride or a carbamate.

It is to be understood that both the foregoing general description and the following detailed description are exemplary, but are not restrictive, of the invention.

### DETAILED DESCRIPTION OF THE INVENTION

Proteins, oligopeptides and polypeptides are polymers of amino acids that have primary, secondary and tertiary structures. The secondary structure of the protein is the local conformation of the polypeptide chain and consists of helices, pleated sheets and turns. The protein's amino acid sequence and the structural constraints on the conformations of the chain determine the spatial arrangement of the molecule. The folding of the secondary structure and the spatial arrangement of the side chains constitute the tertiary structure.

Proteins fold because of the dynamics associated between neighboring atoms on the protein and solvent molecules. The thermodynamics of protein folding and unfolding are defined by the free energy of a particular condition of the protein that relies on a particular model. The process of protein folding involves, amongst other things, amino acid residues packing into a hydrophobic core. The amino acid side chains inside the protein core occupy the same volume as they do in amino acid crystals. The folded protein interior is therefore more like a crystalline solid than an oil drop and so the best model for determining forces contributing to protein stability is the solid reference state.

The major forces contributing to the thermodynamics of protein folding are Van der Waals interactions, hydrogen bonds, electrostatic interactions, configurational entropy and the hydrophobic effect. Considering protein stability, the hydrophobic effect refers to the energetic consequences of removing apolar groups from the protein interior and exposing them to water. Comparing the energy of amino acid hydrolysis with protein unfolding in the solid reference state, the hydrophobic effect is the dominant force. Hydrogen bonds are established during the protein fold process and intramolecular bonds are formed at the expense of hydrogen bonds with water. Water molecules are "pushed out" of the packed, hydrophobic protein core. All of these forces combine and contribute to the overall stability of the folded protein where the degree to which ideal packing occurs determines the degree of relative stability of the protein. The result of maximum packing is to produce a center of residues or hydrophobic core that has maximum shielding from solvent.

Since it is likely that lipophilic drugs would reside in the hydrophobic core of a peptide, it would require energy to unfold the peptide before the drug can be released. The unfolding process requires overcoming the hydrophobic effect by hydrating the amino acids or achieving the melting temperature of the protein. The heat of hydration is a destabilization of a protein. Typically, the folded state of a protein is favored by only 5-15 kcal/mole over the unfolded state. Nonetheless, protein unfolding at neutral pH and at room temperature requires chemical reagents. In fact, partial unfolding of a protein is often observed prior to the onset of irreversible chemical or conformation processes. Moreover, protein conformation generally controls the rate and extent of deleterious chemical reactions.

Conformational protection of active agents by proteins depends on the stability of the protein's folded state and the thermodynamics associated with the agent's decomposition. Conditions necessary for the agent's decomposition should be different than for protein unfolding.

Selection of the amino acids will depend on the physical properties desired. For instance, if increase in bulk or lipophilicity is desired, then the carrier polypeptide will be enriched in the amino acids in the table provided below. Polar amino acids, on the other hand, can be selected to increase the hydrophilicity of the polypeptide.

Ionizing amino acids can be selected for pH controlled peptide unfolding. Aspartic acid, glutamic acid and tyrosine carry a neutral charge in the stomach, but will ionize upon entry into the intestine. Conversely, basic amino acids, such as histidine, lysine and arginine, ionize in the stomach and are neutral in an alkaline environment.

Other factors such as π-π interactions between aromatic residues, kinking of the peptide chain by addition of proline, disulfide crosslinking and hydrogen bonding can all be used to select the optimum amino acid sequence for a given application. Ordering of the linear sequence can influence how these interactions can be maximized and is important in directing the secondary and tertiary structures of the polypeptide.

Furthermore, amino acids with reactive side chains (e.g., glutamic acid, lysine, aspartic acid, serine, threonine and cysteine) can be incorporated for attaching multiple active agents or adjuvants to the same carrier peptide. This is particularly useful if a synergistic effect between two or more active agents is desired.

As stated above, variable molecular weights of the carrier compound can have profound effects on the active agent release kinetics. As a result, low molecular weight active agent delivery systems are preferred. An advantage of this invention is that chain length and molecular weight of the polypeptide can be optimized depending on the level of conformational protection desired. This property can be optimized in concert with the kinetics of the first order release mechanism. Thus, another advantage of this invention is that prolonged release time can be imparted by increasing the molecular weight of the carrier polypeptide. Another, significant advantage of the invention is that the kinetics of active agent release is primarily controlled by the enzymatic hydrolysis of the key bond between the carrier peptide and the active agent.

Dextran is the only polysaccharide known that has been explored as a macromolecular carrier for the covalent binding of drug for colon specific drug delivery. Generally, it was only possible to load up to 1/10 of the total drug-dextran conjugate weight with drug. As stated earlier, polysaccharides are digested mainly in the colon and drug absorption is mainly limited to the colon. As compared to dextran, this invention has two major advantages. First, peptides are hydrolyzed by any one of several aminopeptidases found in the intestinal lumen or associated with the brush-border membrane and so active agent release and subsequent absorption can occur in the jejunum or the ileum. Second, the molecular weight of the carrier molecule can be controlled and, thus, active agent loading can also be controlled.

As a practical example, the following table lists the molecular weights of lipophilic amino acids (less one water molecule) and selected analgesics and vitamins.

**TABLE**

| Amino acid | MW | Active agent | MW |
|---|---|---|---|
| | | | |
| Glycine | 57 | Acetaminophen | 151 |
| Alanine | 71 | Vitamin B₆ (Pyroxidine) | 169 |
| Valine | 99 | Vitamin C (Ascorbic acid) | 176 |
| Leucine | 113 | Aspirin | 180 |
| Isoleucine | 113 | Ibuprofen | 206 |
| Phenylalanine | 147 | Retinoic acid | 300 |
| Tyrosine | 163 | Vitamin B₂ (Riboflavin) | 376 |
| | | Vitamin D₂ | 397 |
| | | Vitamin E (Tocopherol) | 431 |

Lipophilic amino acids are preferred because conformational protection through the stomach is important for the selected active agents, which were selected based on ease of covalent attachment to an oligopeptide. Eighteen was subtracted from the amino acid's molecular weight so that their condensation into a polypeptide is considered. For example, a decamer of glycine (MW=588) linked to aspirin would have a total molecular weight of 750 and aspirin would represent 24% of the total weight of the active agent delivery composition or over two times the maximum drug loading for dextran. This is only for an N- or C- terminus application, for those active agents attached to pendant groups of decaglutamic acid, for instance, a drug with a molecular weight of 180 could conceivably have a loading of 58%, although this may not be entirely practical.

The alcohol, amine or carboxylic acid group of an active agent may be covalently attached to the N-terminus, the C-terminus or the side chain of the oligopeptide or polypeptide. The location of attachment depends somewhat on the functional group selection. For instance, if the active drug is a carboxylic acid (e.g., aspirin) then the N-terminus of the oligopeptide is the preferred point of attachment. If the active agent is an amine (e.g., ampicillin), then the C-terminus is the preferred point of attachment in order to achieve a stable peptide linked active agent. In both, the C- and N-terminus examples, the peptide is, in essence, extended by one monomeric unit forming a new peptide bond. If the active agent is an alcohol, then either the C-terminus or the N-terminus is the preferred point of attachment in order to achieve a stable composition. As in the example above where the alcohol, norethindrone, was covalently attached to poly(hydroxypropylglutamine), an alcohol can be converted into an alkylchloroformate with phosgene. This invention, then, pertains to the reaction of this key intermediate with the N-terminus of the peptide carrier. The active ingredient can be released from the peptide carrier by intestinal peptidases.

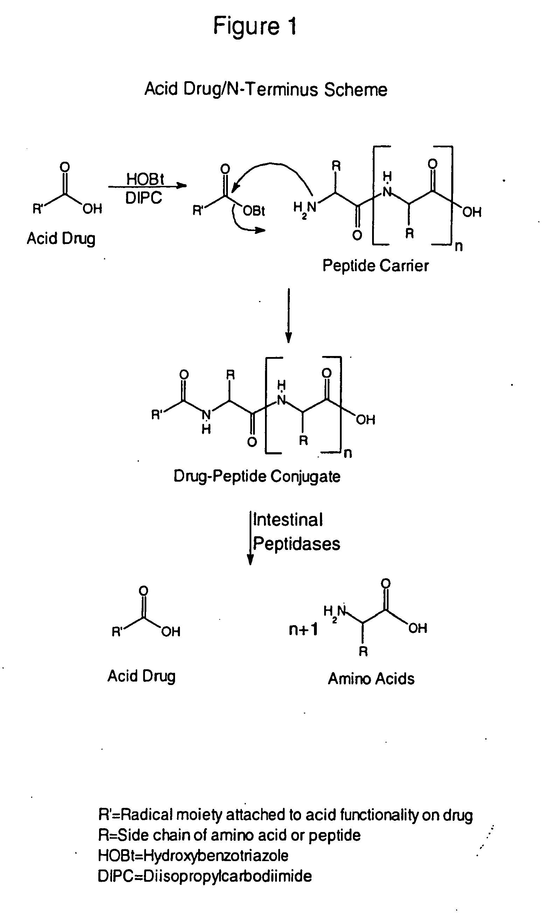

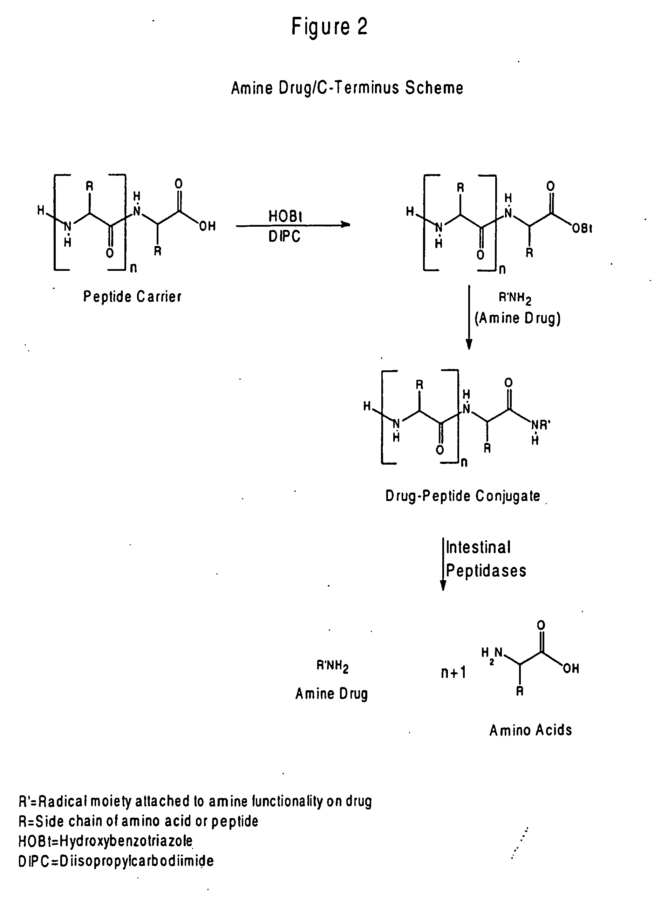

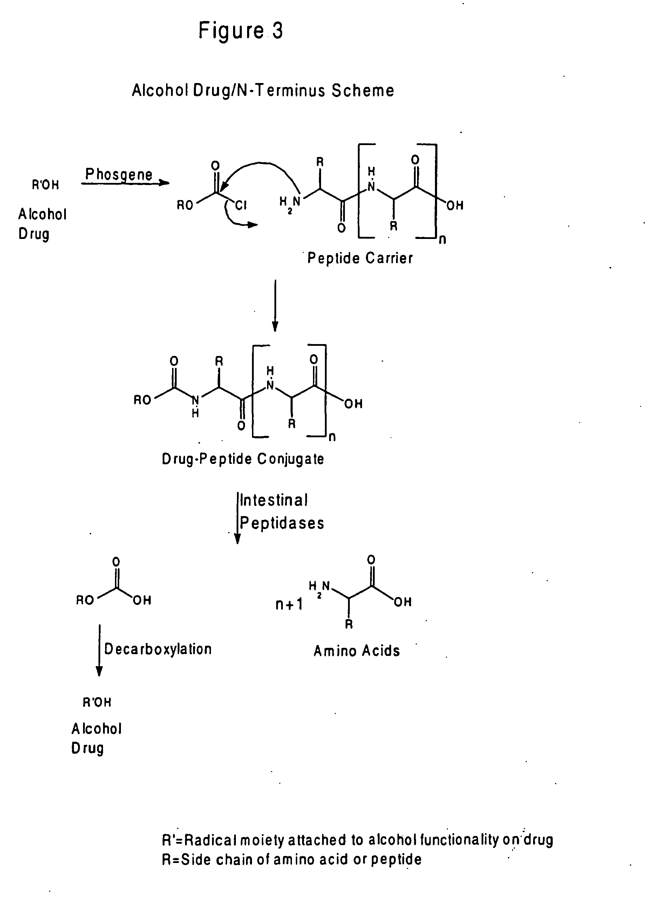

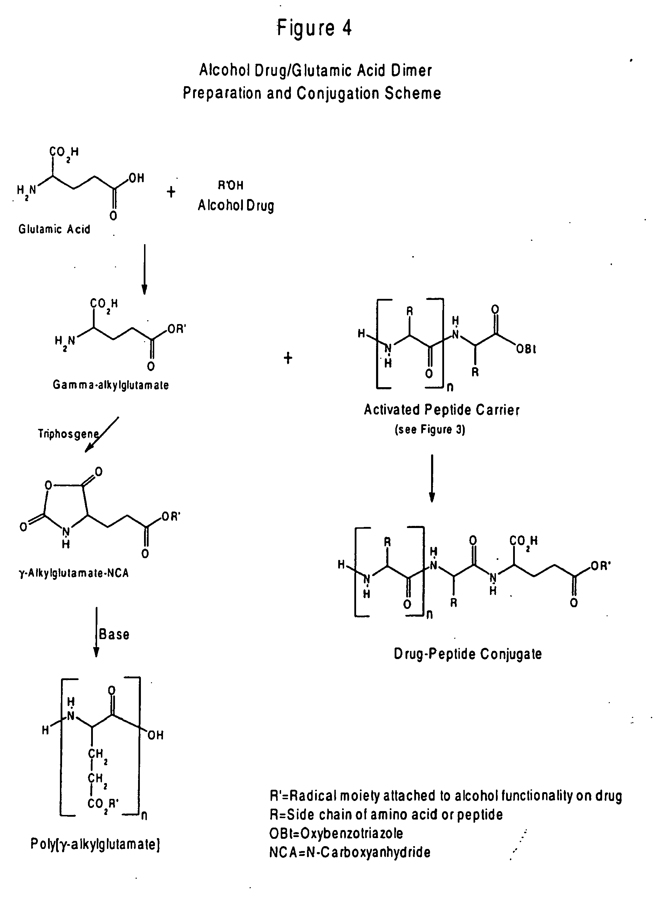

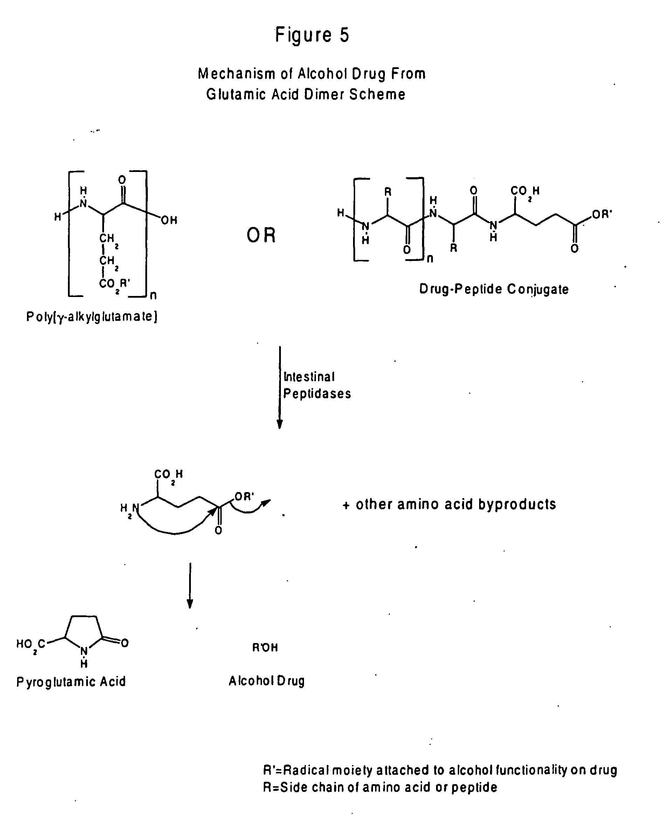

The alcohol can be selectively bound to the gamma carboxylate of glutamic acid and then this conjugate covalently attached to the C-terminus of the peptide carrier. Because the glutamic acid-drug conjugate can be considered a dimer, this product adds two monomeric units to the C-terminus of the peptide carrier where the glutamic acid moiety serves as a spacer between the peptide and the drug as shown in Fig. 4. Intestinal enzymatic hydrolysis of the key peptide bond releases the glutamic acid-drug moiety from the peptide carrier. The newly formed free amine of the glutamic acid residue will then undergo an intramolecular transamination reaction, thereby, releasing the active agent with coincident formation of pyroglutamic acid as shown in Fig. 5. Alternatively, the glutamic acid-drug dimer can be converted into the gamma ester of glutamic acid N-carboxyanhydride. This intermediate can then be polymerized, as described above, using any suitable initiator as shown in Fig. 4. The product of this polymerization is polyglutamic acid with active ingredients attached to multiple pendant groups. Hence, maximum drug loading of the carrier peptide can be achieved. In addition, other amino acid-NCA's can be copolymerized with the gamma ester glutamic acid NCA to impart specific properties to the drug delivery system.

The invention also provides a method of imparting the same mechanism of action for other polypeptides containing functional side chains. Examples include, but are not limited to, polylysine, polyasparagine, polyarginine, polyserine, polycysteine, polytyrosine, polythreonine and polyglutamine. The mechanism can translate to these polypeptides through a spacer or linker on the pendant group, which is terminated, preferably, by the glutamic acid-drug dimer. This carrier peptide-drug conjugate is distinguished from the prior art by virtue of the fact that the primary release of the drug moiety relies on peptidases and not on esterases. Alternatively, the active agent can be attached directly to the pendant group where some other indigenous enzymes in the alimentary tract can affect release.

The active agent can be covalently attached to the N-terminus, the C-terminus or the side chain of the polypeptide using known techniques. Examples of linking organic compounds to the N-terminus type of a peptide include, but are not limited to, the attachment of naphthylacetic acid to LH-RH, coumarinic acid to opioid peptides and 1,3-dialkyl-3-acyltriazenes to tetragastrin and pentagastrin. As another example, there are known techniques for forming peptide linked biotin and peptide linked acridine.

The polypeptide carrier can be prepared using conventional techniques. A preferred technique is copolymerization of mixtures of amino acid N-carboxyanhydrides. Alternatively, if a specific sequence is desired, a solid state automated peptide synthesizer can be used.

The addition of stabilizers to the composition has the potential of stabilizing the polypeptide further. Stabilizers such as sugar, amino acids, polyethylene glycol (PEG) and salts have been shown to prevent protein unfolding. In another embodiment of the invention, a pre-first order release of the active agent is imparted by microencapsulating the carrier polypeptide-active agent conjugate in a polysaccharide, amino acid complex, PEG or salts.

There is evidence that hydrophilic compounds are absorbed through the intestinal epithelia efficiently via specialized transporters. The entire membrane transport system is intrinsically asymmetric and responds asymmetrically to cofactors. Thus, one can expect that excitation of the membrane transport system will involve some sort of specialized adjuvant resulting in localized delivery of active agents. There are seven known intestinal transport systems classified according to the physical properties of the transported substrate. They include the amino acid, oligopeptide, glucose, monocarboxic acid, phosphate, bile acid and the P-glycoprotein transport systems and each has its own associated mechanism of transport. The mechanisms can depend on hydrogen ions, sodium ions, binding sites or other cofactors. The invention also allows targeting the mechanisms for intestinal epithelial transport systems to facilitate absorption of active agents.

In another embodiment of the invention, the composition includes one or more adjuvants to enhance the bioavailability of the active agent. Addition of an adjuvant is particularly preferred when using an otherwise poorly absorbed active agent. Suitable adjuvants, for example, include: papain, which is a potent enzyme for releasing the catalytic domain of aminopeptidase-N into the lumen; glycorecognizers, which activate enzymes in the BBM; and bile acids, which have been attached to peptides to enhance absorption of the peptides.

Compositions of the invention are, in essence, the formation of amides from acids and amines and can be prepared by the following examples.

### Acid/N-terminus conjugation

An acid bioactive agent can be dissolved in DMF under nitrogen and cooled to 0°C. The solution can then be treated with diisopropylcarbodiimide and hydroxybenzotriazole followed by the amine peptide carrier. The reaction can then be stirred for several hours at room temperature, the urea by-product filtered off, the product precipitated out in ether and purified using gel permeation chromatography (GPC) or dialysis.

### Amine/C-terminus conjugation

The peptide carrier can be dissolved in DMF under nitrogen and cooled to 0°C. The solution can then be treated with diisopropylcarbodiimide and hydroxybenzotriazole followed by the amine bioactive agent. The reaction can then be stirred for several hours at room temperature, the urea by-product filtered off, and the product precipitated out in ether and purified using GPC or dialysis.

### Alcohol/N-Terminus Conjugation

In the following example the combination of the alcohol with triphosgene produces a chloroformate, which when reacted with the N-terminus of the peptide produces a carbamate. Pursuant to this, an alcohol bioactive agent can be treated with triphosgene in dry DMF under nitrogen. The suitably protected peptide carrier is then added slowly and the solution stirred at room temperature for several hours. The product is then precipitated out in ether. The crude product is suitably deprotected and purified using GPC.

Other solvents, activating agents, cocatalysts and bases can be used. Examples of other solvents include dimethylsulfoxide, ethers such as tetrahydrofuran or chlorinated solvents such as chloroform. Examples of other activating agents include dicyclohexylcarbodiimide or thionyl chloride. An example of another cocatalyst is N-hydroxysuccinimide. Examples of bases include pyrrolidinopyridine, dimethylaminopyridine, triethylamine or tributylamine.

### Preparation of γ-Alkyl Glutamate

There have been over 30 different γ-alkyl glutamates prepared any one of which may be suitable for the drug alcohol of choice. For example, a suspension of glutamic acid, the alcohol and concentrated hydrochloric acid can be prepared and heated for several hours. The γ-alkyl glutamate product can be precipitated out in acetone, filtered, dried and recrystallized from hot water.

### γ-Alkyl Glutamate/C-Terminus Conjugation

The peptide carrier can be dissolved in DMF under nitrogen and cooled to 0°C. The solution can then be treated with diisopropylcarbodiimide and hydroxybenzotriazole followed by the γ-alkyl glutamate bioactive agent. The reaction can then be stirred for several hours at room temperature, the urea by-product filtered off, and the product precipitated out in ether and purified using GPC or dialysis.

### Preparation of γ-Alkyl Glutamate-NCA

γ-Alkyl glutamate can be suspended in dry THF where triphosgene is added and the mixture refluxed under a nitrogen atmosphere until the mixture becomes homogenous. The solution can be poured into heptane to precipitate the NCA product, which is filtered, dried and recrystallized from a suitable solvent.

### Preparation of Poly[γ-Alkyl Glutamate]

γ-Alkyl glutamate-NCA can be dissolved in dry DMF where a catalytic amount of a primary amine can be added to the solution until it becomes viscous (typically overnight). The product can be isolated from the solution by pouring it into water and filtering. The product can be purified using GPC or dialysis.

The present invention provides several benefits for active agent delivery. First, the invention can stabilize oxycodone and acetaminophen and prevent its digestion in the stomach. In addition, the pharmacologic effect can be prolonged by delayed release of oxycodone and acetaminophen. Furthermore, active agents can be combined to produce synergistic effects. Also, absorption of the active agent in the intestinal tract can be enhanced. The invention also allows targeted delivery of active agents to specifics sites of action.

The composition of the invention comprises oxycodone and acetaminophen covalently attached to a carrier polypeptide. Preferably, the carrier polypeptide is (i) an oligopeptide, (ii) a homopolymer of one of the twenty naturally occurring amino acids, or (iii) a heteropolymer of two or more naturally occurring amino acids,

In the present invention, oxycodone and acetaminophen are covalently attached to the carrier polypeptide via the amino group and hydroxyl group, respectively.

Preferably, the resultant peptide-oxycodone and acetaminophen conjugate is formulated into a tablet using suitable excipients and can either be wet granulated or dry compressed.

The present invention provides several benefits for active' agent delivery. First, the invention can stabilize oxycodone and prevent its digestion in the stomach. In addition, the pharmacologic effect can be prolonged by delayed release of oxycodone. Furthermore, active agents can be combined to produce synergistic effects. Also, absorption of the active agent in the intestinal tract can be enhanced. The invention also allows targeted delivery of active agents to specifics sites of action.

The composition of the invention comprises oxycodone covalently attached to a carrier polypeptide. Preferably, the carrier polypeptide is (i) an oligopeptide, (ii) a homopolymer of one of the twenty naturally occurring amino acids or (iii) a heteropolymer of two or more naturally occurring amino acids.

In the present invention, oxycodone is covalently attached to the carrier polypeptide via the hydroxyl group.

Preferably, the resultant peptide-oxycodone conjugate is formulated into a tablet using suitable excipients and can either be wet granulated or dry compressed.

## Claims

1. A pharmaceutical composition comprising a carrier polypeptide and an active agent covalently attached thereto through a hydroxyl group, wherein:
said active agent is oxycodone;
said polypeptide consists of a homopolymer of one of the twenty naturally occurring amino acids or a heteropolymer of two or more naturally occurring amino acids.

2. The composition of claim 1 wherein said polypeptide is an oligopeptide.

3. The composition of claim 1 wherein said polypeptide is a homopolymer of an amino acid.

4. The composition of claim 1 wherein said polypeptide is a heteropolymer of two or more amino acids.

5. The composition of claim 1 wherein oxycodone is covalently attached to a side chain, the N-terminus or the C-terminus of said polypeptide.

6. The composition of claim 1 further comprising a microencapsulating agent.

7. The composition of claim 6 wherein said microencapsulating agent is selected from the group consisting of polyethylene glycol (PEG), an amino acid, a sugar and a salt.

8. The composition of claim 1 further comprising an adjuvant.

9. The composition of claim 8 wherein said adjuvant activates an intestinal transporter.

10. The composition of claim 1 further comprising a pharmaceutically acceptable excipient.

11. The composition of claim 1 wherein said composition is in the form of a tablet.

12. The composition of claim 1 wherein said composition is in the form of an intravenous preparation.

13. The composition of claim 1 wherein said composition is in the form of an oral suspension.

14. The composition of claim 1 for the treatment of pain.

15. A pharmaceutical composition comprising a carrier polypeptide and an active agent covalently attached thereto through an amino group, wherein:
said active agent is oxycodone;
said carrier polypeptide consists of a homopolymer of one of the twenty naturally occurring amino acids or a heteropolymer of two or more naturally occurring amino acids; and wherein
acetaminophen is present as a further active agent and is covalently bound to the carrier polypeptide via a hydroxyl group.

16. A method for preparing a composition as claimed in claim 1 comprising a polypeptide and oxycodone covalently attached to the polypeptide, the method comprising the steps of:
(a) attaching oxycodone to a side chain of an amino acid to form an oxycodone/amino acid complex;
(b) forming an oxycodone/amino acid complex N-carboxyanhydride (NCA) from the oxycodone/amino acid complex; and
(c) polymerizing the oxycodone/amino acid complex N-carboxyanhydride (NCA).

## Patentansprüche

1. Pharmazeutische Zusammensetzung, die ein Trägerpolypeptid und ein aktives Mittel umfasst, das über eine Hydroxylgruppe kovalent daran gebunden ist, wobei:
es sich bei dem genannten aktiven Mittel um Oxycodon handelt;
das genannte Polypeptid ein Homopolymer einer der zwanzig natürlich vorkommenden Aminosäuren oder ein Heteropolymer aus zwei oder mehr natürlich vorkommenden Aminosäuren umfasst.

2. Zusammensetzung nach Anspruch 1, wobei es sich bei dem genannten Polypeptid um ein Oligopeptid handelt.

3. Zusammensetzung nach Anspruch 1, wobei es sich bei dem genannten Polypeptid um ein Homopolymer einer Aminosäure handelt.

4. Zusammensetzung nach Anspruch 1, wobei es sich bei dem genannten Polypeptid um ein Heteropolymer aus zwei oder mehr Aminosäuren handelt.

5. Zusammensetzung nach Anspruch 1, wobei Oxycodon kovalent gebunden ist an eine Seitenkette, den N-Terminius oder den C-Terminus des genannten Polypeptids.

6. Zusammensetzung nach Anspruch 1, wobei diese ferner ein Mikroverkapselungsmittel umfasst.

7. Zusammensetzung nach Anspruch 6, wobei das genannte Mikroverkapselungsmittel ausgewählt wird aus der Gruppe, die Polyethylenglykol (PEG), eine Aminosäure, einen Zucker und ein Salz umfasst.

8. Zusammensetzung nach Anspruch 1, wobei diese ferner einen Hilfsstoff umfasst.

9. Zusammensetzung nach Anspruch 8, wobei der genannte Hilfsstoff einen Darmtransportstoff aktiviert.

10. Zusammensetzung nach Anspruch 1, wobei diese ferner einen pharmazeutisch zulässigen Arzneistoffträger umfasst.

11. Zusammensetzung nach Anspruch 1, wobei die genannte Zusammensetzung in Form einer Tablette vorgesehen ist.

12. Zusammensetzung nach Anspruch 1, wobei die genannte Zusammensetzung in Form einer intravenösen Präparation vorgesehen ist.

13. Zusammensetzung nach Anspruch 1, wobei die genannte Zusammensetzung in Form einer oralen Suspension vorgesehen ist.

14. Zusammensetzung nach Anspruch 1 zur Schmerzbehandlung.

15. Pharmazeutische Zusammensetzung, die ein Trägerpolypeptid und ein aktives Mittel umfasst, das über eine Aminogruppe kovalent daran gebunden ist, wobei:
es sich bei dem genannten aktiven Mittel um Oxycodon handelt;
das genannte Trägerpolypeptid ein Homopolymer einer der zwanzig natürlich vorkommenden Aminosäuren oder ein Heteropolymer aus zwei oder mehr natürlich vorkommenden Aminosäuren umfasst; und wobei
Acetaminophen als ein weiteres aktives Mittel vorhanden und über eine Hydroxylgruppe kovalent an das Trägerpolypeptid gebunden ist.

16. Verfahren zur Herstellung einer Zusammensetzung nach Anspruch 1, wobei dieses ein Polypeptid und Oxycodon umfasst, das kovalent an das Polypeptid gebunden ist, wobei das Verfahren die folgenden Schritte umfasst:
(a) das Binden von Oxycocon an eine Seitenkette einer Aminosäure, so dass ein Oxycodon/Aminosäurekomplex gebildetwird;
(b) das Bilden eines Oxycodon/Aminosäurekomplex-N-Carboxyanhydrids (NCA) aus dem Oxycodon/Aminosäurekomplex; und
(c) das Polymerisieren des Oxycodon/Aminosäurekomplex-N-Carboxyanhydrids (NCA).

## Revendications

1. Composition pharmaceutique comprenant un polypeptide support et un agent actif lié par covalence à celui-ci par un groupe hydroxyle, dans laquelle :
ledit agent actif est l'oxycodone ;
ledit polypeptide est constitué d'un homopolymère d'un des vingt acides aminés se produisant naturellement ou d'un hétéropolymère de deux acides aminés se produisant naturellement ou plus.

2. Composition selon la revendication 1, dans laquelle ledit polypeptide est un oligopeptide.

3. Composition selon la revendication 1, dans laquelle ledit polypeptide est un homopolymère d'un acide aminé.

4. Composition selon la revendication 1, dans laquelle ledit polypeptide est un hétéropolymère de deux acides aminés ou plus.

5. Composition selon la revendication 1, dans laquelle l'oxycodone est liée par covalence à une chaîne latérale, la terminaison N ou la terminaison C dudit polypeptide.

6. Composition selon la revendication 1, comprenant en outre un agent de microencapsulation.

7. Composition selon la revendication 6, dans laquelle ledit agent de microencapsulation est sélectionné parmi le groupe constitué du polyéthylèneglycol (PEG), d'un acide aminé, d'un sucre et d'un sel.

8. Composition selon la revendication 1, comprenant en outre un adjuvant.

9. Composition selon la revendication 8, dans laquelle ledit adjuvant active un transporteur intestinal.

10. Composition selon la revendication 1, comprenant en outre un excipient pharmaceutiquement acceptable.

11. Composition selon la revendication 1, dans laquelle ladite composition est sous la forme d'un comprimé.

12. Composition selon la revendication 1, dans laquelle ladite composition est sous la forme d'une préparation intraveineuse.

13. Composition selon la revendication 1, dans laquelle ladite composition est sous la forme d'une suspension orale.

14. Composition selon la revendication 1 pour le traitement de la douleur.

15. Composition pharmaceutique comprenant un polypeptide support et un agent actif lié par covalence à celui-ci par un groupe amino, dans laquelle :
ledit agent actif est l'oxycodone ;
ledit polypeptide support est constitué d'un homopolymère d'un des vingt acides aminés se produisant naturellement ou d'un hétéropolymère de deux acides aminés se produisant naturellement ou plus ; et dans laquelle
l'acétaminophène est présent comme autre agent actif et est lié par covalence au polypeptide support par le biais d'un groupe hydroxyle.

16. Procédé de préparation d'une composition selon la revendication 1 comprenant un polypeptide et de l'oxycodone liée par covalence au polypeptide, le procédé comprenant les étapes consistant à :
(a) lier l'oxycodone à une chaîne latérale d'un acide aminé pour former un complexe oxycodone/acide aminé ;
(b) former un N-carboxyanhydride (NCA) de complexe oxycodone/acide aminé à partir du complexe oxycodone/acide aminé ; et
(c) polymériser le N-carboxyanhydride (NCA) de complexe oxycodone/acide aminé.
